# EUROPÄISCHE PATENTANMELDUNG

(11) **EP 2 813 200 A1**
(43) Veröffentlichungstag der Anmeldung: **17.12.2014**
(21) Anmeldenummer: 13005630.2
(22) Anmeldetag: 04.12.2013
(51) Int. Cl.: A61F 13/532, A61F 13/15, B32B 5/16, B32B 7/04, B32B 7/06, B32B 7/14, B32B 27/14, B32B 3/10, A61F 13/53

(54) **Absorbierende Komposite**

(30) Priorität: 14.06.2013 EP 13003046
(71) Anmelder: RKW SE, 67227 Frankenthal (DE)
(72) Erfinder: Kirsch, Andreas, 31167 Bockenem (DE); Thelen, Bernd, 53844 Troisdorf (DE); Krotzek, Matthias, 06484 Quedlinburg (DE)
(74) Vertreter: Wagner, Jutta

(57) **Zusammenfassung**

Die vorliegende Erfindung betrifft absorbierende Komposite enthaltend Superabsorber-Partikel und mindestens zwei Materiallagen, wobei die Superabsorber-Partikel zwischen zwei Materiallagen so positioniert werden, dass Bereiche mit Superabsorber-Partikeln und Bereiche ohne Superabsorber-Partikel gebildet werden, und die Materiallagen in den Bereichen ohne Superabsorber-Partikel miteinander verbunden sind, so dass die zwei Materiallagen aus den Bereichen mit Superabsorber-Partikeln zumindest eine Kammer bilden, in welcher die Superabsorber-Partikel eingeschlossen sind. Weiterhin wird die Aufgabe durch ein Verfahren gelöst, bei dem mindestens zwei Materiallagen bereitgestellt werden, Superabsorber-Partikel zwischen die beiden Materiallagen bereichsweise eingebracht werden und die Materiallagen in den Bereichen ohne Superabsorber-Partikel miteinander verbunden werden, wobei die beiden Materiallagen in den Bereichen mit Superabsorber-Partikeln zumindest eine Kammer ausbilden, in der die Superabsorber-Partikel eingeschlossen sind.

## Beschreibung

Die vorliegende Erfindung betrifft absorbierende Komposite, bei denen absorbierende Partikel in mindestens eine von Materiallagen gebildete Kammer eingeschlossen sind, und Verfahren zu ihrer Herstellung.

Absorbierende Komposite werden heutzutage in den unterschiedlichsten Anwendungsbereichen eingesetzt. Zu nennen sind hier vor allem der Bereich Hygiene (Windeln, Slipeinlagen, Medizin, Inkontinenzunterlagen), der Lebensmittelbereich (Meat pads), technische Anwendungen (Kabelummantelungen) und Verpackungen (Korrosionsschutz).

Eine Hauptaufgabe der absorbierenden Komposite besteht darin, eine definierte Absorptionskapazität zielgenau zu positionieren und dabei die bestmögliche Funktionalität des Absorptionsmediums zu gewährleisten. Als Absorptionsmedium dienen häufig sog. Superabsorber, das sind in der Regel Polymere, welche ein vielfaches ihres Eigengewichts bzw. -volumens an Flüssigkeit aufnehmen und binden können. Außerdem ist eine effiziente und einfache Verarbeitung wichtig.

In der Hygieneindustrie werden Absorbent-Core Konzepte mit Hilfe von zellstoffbasierten Faserstrukturen in Kombination mit superabsorbierenden Körnern oder Fasern gebildet oder mit Hilfe von Superabsorber-Körnern, die auf eine Materiallage, beispielsweise eine Pulplage, ein Airlaidmaterial oder ein Vliesmaterial aufgestreut, fixiert und danach mit einer weiteren Lage bedeckt werden.

Grundsätzlich kann der Eintrag der superabsorbierenden Bestandteile in Form von Fasern oder in Form von Körnern erfolgen. Bei der Fasereinbringung wird normalerweise eine gleichmäßige Absorptionskapazität in der gesamten Fläche erzielt, eine zielgerichtete Konzentration der Absorptionskapazität an bestimmten Stellen ist nicht möglich. Ein weiterer Nachteil sind die Kosten der Fasern, die Absorptionskapazität gemessen in Litern pro eingesetztem Euro ist bei den superabsorbierenden Fasern wesentlich geringer, als bei den superabsorbierenden Körnern.

Die Superabsorber-Körner können eingebracht werden, indem sie bei der Formung eines Pads/Flächengebildes aus Zellulosefasern zielgenau an bestimmten Stellen eingeblasen und damit zugleich positioniert werden. Eine weitere Methode besteht darin, Superabsorber-Körner auf Materiallagen aufzustreuen, nötigenfalls mit Kleber zu fixieren, und dann mit einer weiteren Materiallage abzudecken. Nachteilig an dieser Vorgehensweise ist der relativ große Aufwand.

Im Lebensmittelbereich werden absorbierende Komposite bei der Verpackung von Lebensmitteln eingesetzt, um austretende Flüssigkeiten aufzusaugen und zu speichern, sog. Meat pads. Die heute überwiegend eingesetzten Meat Pads bestehen vornehmlich aus kurzfaserigen Pulpfasern (Airlaid-Material), die mittels des Airlaid-Verfahrens, in bestimmten Materialvarianten auch in Kombination mit Superabsorber-Pulver oder -Fasern, hergestellt werden.

Ein gravierender Nachteil bei der Verwendung der Meat Pads besteht darin, dass wenn es mit dem zu verpackenden Lebensmittel in Kontakt tritt, sich die Fasern aus dem Produkt lösen und an dem Lebensmittel haften bleiben. Ein zweiter wesentlicher Nachteil ist, dass beim Verarbeiten von Rollenware ein Faserstaub beim Abwickeln der Rolle und beim Schneiden der einzelnen Pads entsteht, so dass in kurzen temporären Abständen die Produktion abgestellt werden muss, um den gesammelten Schneidstaub zu entfernen. Dies reduziert die Maschineneffizienz signifikant.

Darüber hinaus hat das geschnittene Pad vier offene Seiten (von der Schnittkante her gesehen). Beim Aufsaugen der aus dem Lebensmittel austretenden Flüssigkeit expandieren die Superabsorber-Fasern bzw. -Pulver und treten dadurch an den vier offenen Seiten aus. Eine Verschweißung dieser Kanten ist nicht möglich, da das Produkt aus kurzfaserigen Zellstoff/Pulpfasern hergestellt wird. Um den Austritt der Fasern beim jetzt eingesetzten Produkt zu verhindern, wird das gesamte Pad in eine so genannte Folientasche komplett eingeschweißt. Dies bedeutet einen zusätzlichen Arbeitsschritt und somit eine Erhöhung des Preises der Pads.

Hauptsächlich werden für Meat Pads Superabsorber-Fasern eingesetzt. Diese Absorberfasern werden zu Zeit nur von einem einzigen Produzenten in England hergestellt. Hier besteht das Risiko bei Produktionsausfall oder Rohstoffverknappung, dass eine kontinuierliche Belieferung dadurch nicht gewährleistet ist. Darüber hinaus ist die Preissituation von der Monopolsituation gekennzeichnet.

Die Nachteile der derzeitigen Verfahren sind die nicht hinreichende Bestimmung der Absorptionskapazität, die nicht hinreichend präzise Positionierung der Absorptionskapazität sowie die komplexen, komplizierten und kostentreibenden Verarbeitungsschritte, um derartige Produkte herzustellen.

Es besteht daher die Aufgabe, eine genau definierte Absorptionskapazität präzise positioniert und leicht verarbeitbar in Rollenform bereitzustellen, um eine effiziente, kostengünstige und unkomplizierte Weiterverarbeitung zu garantieren.

Überraschend wurde nun gefunden, dass es möglich ist, einen Superabsorber in Pulver- oder Körnerform zwischen zwei Materiallagen, vorzugsweise zwischen zwei Vlieslagen, insbesondere zwei Lagen aus wasserstrahlverfestigtem Vlies, einzubringen, wobei der Superabsorber örtlich genau definiert eingebracht wird und die Vlieslagen durch Verschweißung zumindest eine komplett geschlossene Kammer bilden, so dass der Superabsorber dauerhaft positioniert ist.

Die obige Aufgabe wird daher gelöst durch ein absorbierendes Komposit enthaltend Superabsorber-Partikel und mindestens zwei Materiallagen, wobei die Superabsorber-Partikel zwischen die zwei Materiallagen so positioniert sind, dass Bereiche mit Superabsober-Partikeln und Bereiche ohne Superabsorber-Partikel gebildet werden, und die Materiallagen in den Bereichen ohne SuperabsorberPartikel miteinander verbunden sind, so dass die zwei Materiallagen aus den Bereichen mit Superabsorber-Partikeln mindestens eine Kammer bilden, in welchen die Superabsorber-Partikel eingeschlossen sind. Weiterhin wird die Aufgabe durch ein Verfahren gelöst, bei dem mindestens zwei Materiallagen bereitgestellt werden, Superabsorber-Partikel zwischen die beiden Materiallagen bereichsweise eingebracht werden und die Materiallagen in den Bereichen ohne Superabsorber-Partikel miteinander verbunden werden, wobei die Materiallagen in den Bereichen mit Superabsorber-Partikeln mindestens eine Kammer ausbilden, in denen die Superabsorber-Partikel eingeschlossen sind.

Bei der erfindungsgemäßen Herstellung der Komposite wird vorzugsweise eine erste gas- und/oder flüssigkeitsdurchlässige Materiallage bereit gestellt. Der Superabsorber wird auf der Materiallage positioniert. Die zweite Materiallage wird zugeführt und anschließend, bevorzugt in einem gleichen Arbeitsschritt, verschweißt. Größe und Form der Kammer bzw. Kammern können hierbei flexibel gestaltet werden. Mit dem erfindungsgemäßen Verfahren können somit unterschiedliche Saugleistungen in unterschiedlichen Formen und Größen erzeugt werden. Das erfindungsgemäße Komposit kann in unterschiedlichsten Applikationen und Bereichen eingesetzt werden. Auch besteht die Möglichkeit unterschiedliche Materiallagen einzusetzen, vorausgesetzt, dass zumindest die Lagen, durch die Flüssigkeit absorbiert werden soll, flüssigkeitsdurchlässig sind.

Ganz wesentlich bei den erfindungsgemäßen Kompositen und Verfahren ist, dass der Superabsorber genau positioniert und auch in der Menge geregelt werden kann. Die Positionierung erfolgt z.B. mit Hilfe einer Präzisionsstreumaschine, wie sie unter anderem von der Firma TPS TechnoPartner Samtronic GmbH, DE, erhältlich ist. In einer bevorzugten Variante kann der Superabsorber so positioniert werden, dass keine Partikel in die Bereiche gelangen, in denen die Materiallagen zur Bildung der Kammern miteinander verbunden werden. Auch kann die in der einzelnen Kammer positionierte Menge der Superabsorber-Partikel und damit die Absorptionskapazität gesteuert werden. Darüber hinaus ist bei geeigneter Wahl von Superabsorbermenge und Kammergröße gewährleistet, dass der Superabsorber seine Absorptionswirkung vollständig entfalten kann, indem er ungehindert quillt und ein Gel-Blocking verhindert wird.

Als Superabsorber eignen sich besonders Superabsorber-Körner und -Pulver, aber auch entsprechend kurze Superabsorber-Fasern. Erfindungsgemäß kann sowohl die Menge als auch die Lage der Superabsorber-Partikel genau gesteuert werden. Superabsorber-Materialien sind an sich bekannt und erfindungsgemäß nicht eingeschränkt. Sie werden in an sich bekannter Weise entsprechend der Anwendung gewählt, beispielsweise hinsichtlich der zu absorbierenden Flüssigkeit, deren Menge usw. Es ist ein großer Vorteil, dass sich die Absorptionskapazität erfindungsgemäß auch bei Körnern und Pulvern einstellen und sehr exakt positionieren lässt.

Als Materiallagen kommen im Prinzip alle Arten von verschweißbaren Flächengebilden in Betracht. Vorzugsweise Vliese und Folien, besonders bevorzugt Vliese. Die Materiallagen können gleich oder verschieden sein. Es können mehr als zwei Materiallagen verwendet werden.

Erfindungsgemäß sind Vliese als Materiallage bevorzugt. Es können alle bekannten Vliese eingesetzt werden, vorzugsweise Vliese aus Stapelfasern oder Endlosfilamenten. Als Material für die Vlieslagen kommen Stapel- und Endlosfasern in Betracht, sowohl Polymerfasern als auch andere verschweißbare Fasern und natürliche und halbsynthetische Fasern. Fasergemische sind ebenso möglich, beispielsweise können einem Spinnvlies aus Endlosfasern Naturfasern beigemischt werden. In jedem Fall muss der Anteil an verschweißbaren Fasern und/oder Polymerfasern so hoch sein, dass die Verbindung der Vlieslagen zur Bildung der Kammern durch Verschweißung, z.B. mittels Druck oder Wärme und Druck oder per Ultraschall, erfolgen kann.

Das erfindungsgemäße absorbierende Komposit weist mindestens eine Kammer auf, vorzugsweise weist es zwei, drei, vier oder noch mehr Kammern auf. Die Anzahl, Form und Anordnung der Kammern richtet sich nach der Anwendung. Bei Kompositen, die auch nach der Flüssigkeitsaufnahme flach bleiben sollen, bewähren sich kleinere und zahlreichere Kammern. Für eine möglichst große Kapazität sind dagegen weniger aber größere Kammern besser geeignet. Die Kammern in einem Komposit können sich in Form und Größe unterscheiden. Typischerweise haben die Kammern unabhängig voneinander Flächen im Bereich von 5 x 5 mm² bis 100 x 140 mm².

Das erfindungsgemäß absorbierende Komposit weist mindestens eine Kammer auf, die vorzugsweise rundum geschlossen ist. Zur besseren, optimalen Auslastung der Saugkapazität kann die Fläche der Kammer(n) mit einer oder mehreren Hilfsverschweißung(en) versehen sein. Diese dient dazu, dass der auf der Materiallage positionierte Superabsorber fixiert wird und auch für den Transport in der Position gesichert ist.

Die Hilfsverschweißung dient zur optimalen Superabsorber Fixierung und ermöglicht ein gleichmäßiges Quellen in der (den) Kammer(n). Unter dem gleichmäßigen Quelldruck kann sich die Hilfsverschweißung öffnen, somit wird eine noch bessere und höhere Absorbtionsleistung des Komposits ermöglicht.

In einer bevorzugten Ausführungsform ist zumindest eine Materiallage ein Spinnvlies, vorzugsweise ein wasserstrahlverfestigtes Spinnvlies.

Insbesondere im Anwendungsbereich Meat Pads wird bei Einsatz von einem Spinnvlies, idealerweise einem wasserstrahlverfestigten Vlies aus Endlosfilamenten, durch die Endlosfilamente verhindert, dass sich einzelne Fasern, wie bei Pulp- oder Airlaidmaterial, herauslösen und am zu verpackenden Lebensmittel anlagern können. Durch die Rundumschweißung der Kammern des Pads wird zudem verhindert, dass Superabsorber an den Rändern austreten kann. Darüber hinaus entsteht beim Abrollen und beim Schneiden der einzelnen Pads kein Schneidstaub wodurch eine höhere Effizienz der Produktionsanlage erzielt wird.

Die Erfindung soll anhand der folgenden Beispiele erläutert werden, ohne jedoch auf die speziell beschriebenen Ausführungsformen beschränkt zu sein. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

### Beispiel 1

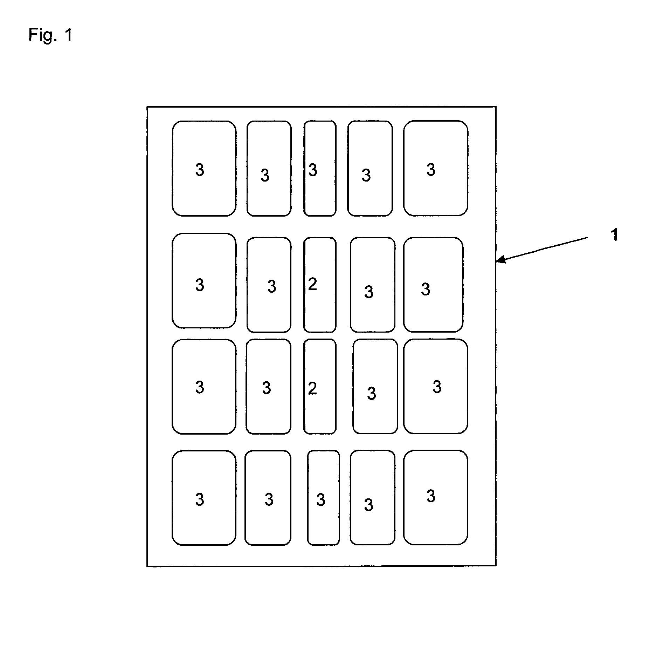

Es wurden Saugpads mit einer Größe von 90mm x 135mm gefertigt. Die Kammern in dem Pad 1 wurden wie in Figur 1 gezeigt erzeugt, wobei in der mittleren Kammer 2 kein Superabsorber und in den äußeren Kammern 3 ein Gesamtmenge von ca. 0,3 g Superabsorber enthalten waren. Zur Simulation der Gewebeflüssigkeit wurde isotonische Kochsalzlösung eingesetzt (0,9 %-ige NaCl-Lösung). Als Superabsorber wurde FAVOR PAC 300 (EVONIK Stockhausen GmbH, DE) ausgewählt. Bei diesem Superabsorber liegt die Wasseraufnahme laut Datenblatt bei ca. 77 g/g und die Aufnahme von 0,9 %-ige NaCl-Lösung bei ca. 44 g/g. Als Vliesstoff wurde wasserstrahlverfestigtes Polypropylen-Spinnvlies (Endlosfasern) Hydrospun / HY-Jet /HS 242 70g/m² hydrophil ausgerüstet (RKW SE, DE) eingesetzt. Das Gewicht des Vliesstoffes bei einer Größe von 135 mm x 90 mm, bei doppelter Lage, lag bei ca. 1,8 g (theoretisch bei 1,7 g). Der Vliesstoff hielt ca. 10 g Flüssigkeit in seinen Fasen fest, so dass ein mit Flüssigkeit getränktes Pad ca. 12 g Eigengewicht aufwies. Die Art der Flüssigkeit (Wasser oder 0,9 %-ige NaCl-Lösung) spielte dabei eine untergeordnete Rolle.

Ein Pad, welches mit Wasser getränkt wurde, wies ein maximales Gesamtgewicht von ca. 30 g auf, zusammengesetzt aus 12 g getränkter Vliesstoff und 18 g Wasseraufnahme durch den Superabsorber. Die Zeit bis zur maximalen Sättigung betrug ca. 15 - 20 Minuten. Wurde das Pad mit 0,9 %-iger NaCl-Lösung getränkt, wurde ein maximales Gesamtgewicht von 25 g erreicht, zusammengesetzt aus 12 g getränkter Vliesstoff und 13 g Aufnahme 0,9 %-ige NaCl-Lösung durch den Superabsorber. Die Zeit bis zur maximalen Sättigung betrug hier ca. 20 - 30 Minuten. Die Kammern des Pads waren nicht so prall gefüllt, wie bei den Tränkversuchen mit Wasser. Die Kammern ließen sich durch Verschweißen problemlos herstellen und es trat weder beim Schneiden noch beim Quellen Superabsorber aus.

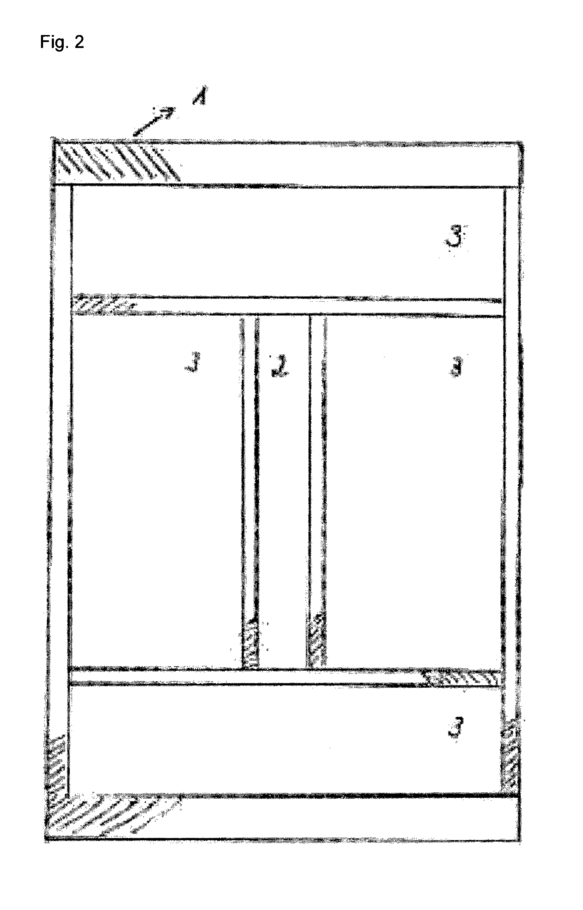

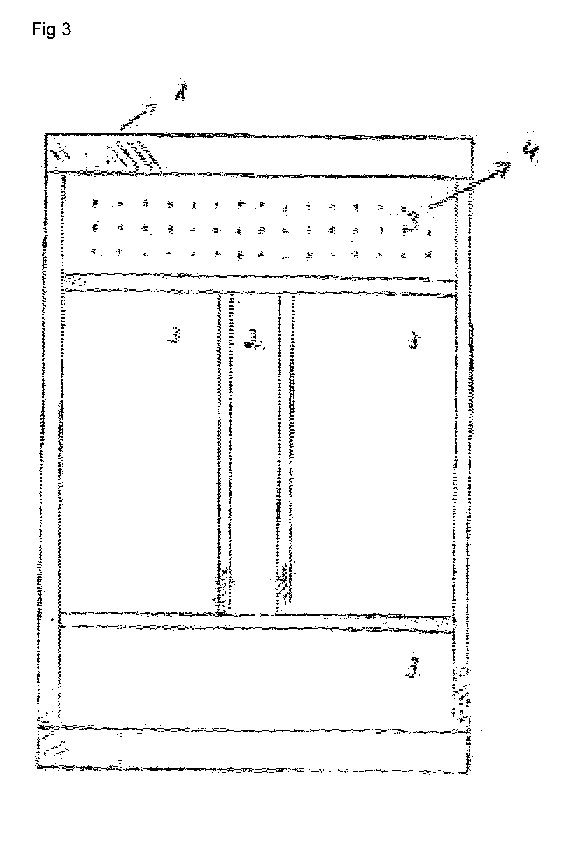

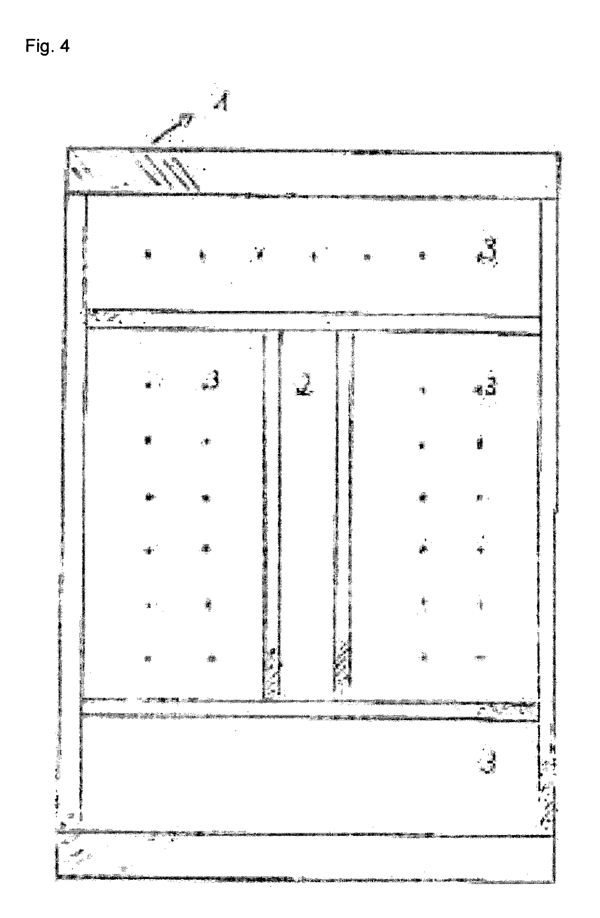

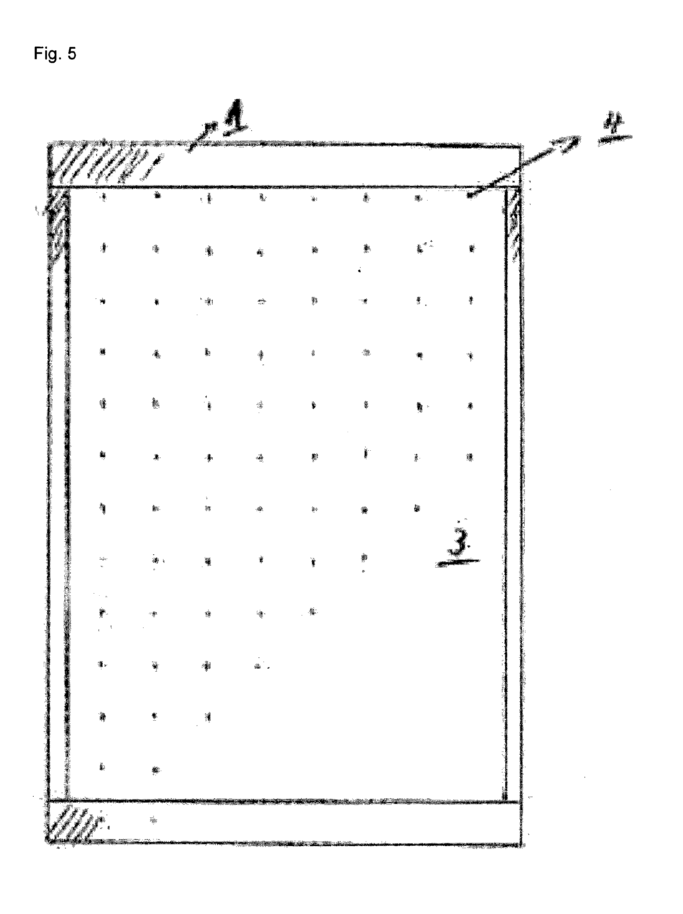

Die Figuren 2 bis 5 zeigen Varianten mit unterschiedlichen Kammergrößen für verschiedene Anwendungszwecke. Bei der Ausführungsformen in Figuren 3, 4 und 5 sind in einigen Kammern 3 Hilfsverschweißungen 4 vorgenommen worden. Diese tragen dazu bei, den Superabsorber zusätzlich in seiner Lage zu fixieren, auch innerhalb der Kammer. Die Temperatur beim Schweißen wird so geregelt, dass die Hilfsverschweißungen sich lösen, wenn sich der Superabsorber durch die Flüssigkeitsaufnahme ausdehnt.

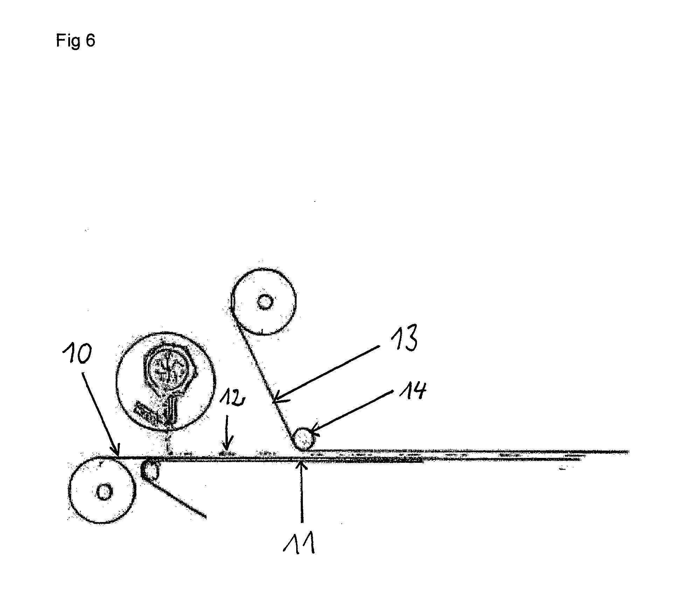

Das erfindungsgemäße Verfahren ist in den Figuren 6, 7 und 8 schematisch veranschaulicht. Wie in Figur 6 gezeigt, wird eine erste Materiallage, der Träger 10 von einer Rolle abgewickelt und einem Transportband 11 zugeführt. Auf dieser liegend wird Superabsorber 12 bereichsweise aufgebracht. Dann wird eine zweite Materiallage, Abdeckung 13 von oben aufgelegt. Die Umlenk- und Andruckwalze 14 kompaktiert die Lagen.

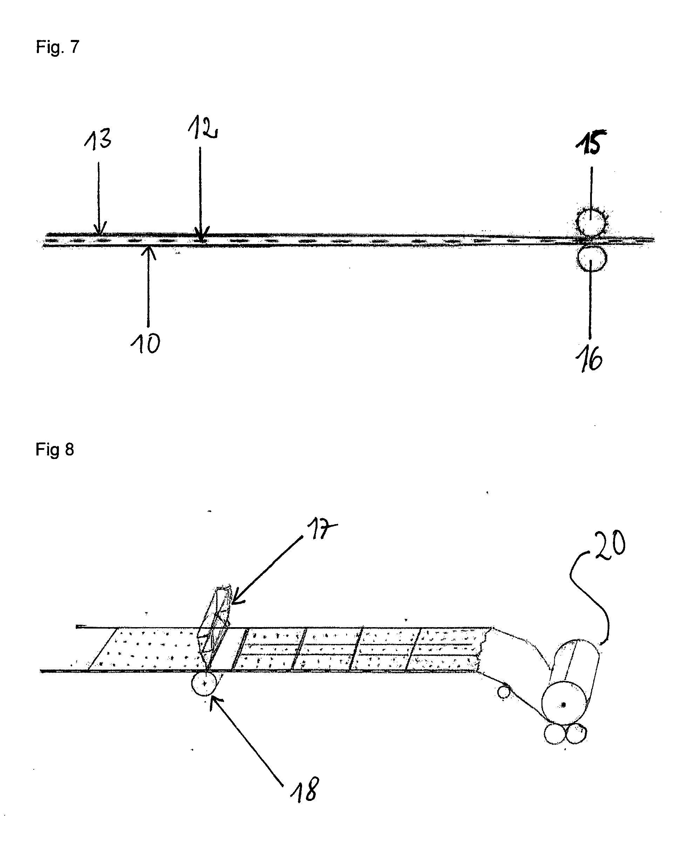

Sofern eine Hilfsverschweißung 4 erfolgen soll, werden die kompaktierten Lagen 10 und 13 mit dem dazwischenliegenden Superabsorber 12 einer thermischen Bondierung mittels Kalanderwalzen 15, 16 unterzogen, wie in Figur 7 gezeigt.

Zur Ausbildung der Kammern werden die kompaktierten Lagen, mit oder ohne Hilfsverschweißung 4, einer Ultraschallschweißung unterworfen. Dazu laufen die Lagen durch eine Ultraschallschweißeinheit aus Sonotrode/Amboss 17 und Gravurwalze 18. Das fertige absorbierende Komposit wird in Form von Rollenware 20 erhalten.

## Patentansprüche

1. Absorbierendes Komposit (1) enthaltend Superabsorber-Partikel (12) und mindestens zwei Materiallagen (10, 13), wobei die Superabsorber-Partikel (12) zwischen die zwei Materiallagen (10, 13) so positioniert werden, dass Bereiche mit Superabsober-Partikeln und Bereiche ohne Superabsorber-Partikel ausgebildet sind, und die Materiallagen (10, 13) in den Bereichen ohne Superabsorber-Partikel miteinander verbunden sind, so dass die zwei Materiallagen (10, 13) aus den Bereichen mit Superabsorber-Partikeln mindestens eine Kammer (3) bilden, in welchen die Superabsorber-Partikel eingeschlossen sind.

2. Komposit gemäß Anspruch 1, **dadurch gekennzeichnet, dass** die Materiallagen (10, 13) durch Verschweißung, beispielsweise mittels Druck oder Wärme und Druck oder mittels Ultraschall, miteinander verbunden sind.

3. Komposit gemäß Anspruch 1 oder 2, **dadurch gekennzeichnet, dass** die Materiallagen (10, 13) unabhängig voneinander ausgewählt sind unter Vlieslagen, Folienlagen, Geweben und Gewirken oder ähnlichen Flächengebilden.

4. Komposit gemäß Anspruch 3, **dadurch gekennzeichnet, dass** vorzugsweise eine Materiallage (10) ein Vlies ist, insbesondere ein Spinnvlies oder ein wasserstrahlverfestigtes Endlosfilamentvlies ist.

5. Komposit gemäß Anspruch 3, **dadurch gekennzeichnet, dass** zumindest eine Materiallage (10, 13) gas- und/oder flüssigkeitsdurchlässig ist.

6. Komposit gemäß einem der Ansprüche 1 bis 5, **dadurch gekennzeichnet, dass** die Materiallagen (10, 13), unabhängig voneinander, aus verschweißbaren Rohstoffen, bestehen.

7. Komposit gemäß einem der Ansprüche 1 bis 6, **dadurch gekennzeichnet, dass** zumindest zwei, vorzugsweise drei, vier, fünf, sechs, acht, zehn oder zwölf Kammern (2, 3) gebildet sind.

8. Komposit gemäß einem der Ansprüche 1 bis 7, **dadurch gekennzeichnet, dass** die Superabsorberpartikel (12) Superabsorber-Körner und/oder Superabsorber-Pulver sind.

9. Verfahren zur Herstellung absorbierender Komposite (1), enthaltend Superabsorber-Partikel (12) und mindestens zwei Materiallagen (10, 13), **dadurch gekennzeichnet, dass** die mindestens zwei Materiallagen (10, 13) bereitgestellt werden, Superabsorber-Partikel (12) zwischen die beiden Materiallagen (10, 13) bereichsweise eingebracht werden und die Materiallagen (10, 13) in den Bereichen ohne Superabsorber-Partikel miteinander verbunden werden, wobei die Materiallagen (10, 13) in den Bereichen mit Superabsorber-Partikeln (12) zumindest eine Kammer (3) ausbilden, in der die Superabsorber-Partikel (12) eingeschlossen sind.

10. Verfahren gemäß Anspruch 9, **dadurch gekennzeichnet, dass** diese Materiallagen (10, 13) aus Polymeren hergestellt sind und durch Verschweißen, beispielsweise mittels Druck oder Wärme und Druck oder mittels Ultraschall, miteinander verbunden werden.

11. Verfahren gemäß Anspruch 9 oder 10, **dadurch gekennzeichnet, dass** die Superabsorberpartikel (12) auf einer ersten Materiallage (10) positioniert werden und mit einer zweiten Materiallage (13) abgedeckt werden, wobei die Materiallagen (10, 13) in dem gleichen Arbeitsschritt miteinander verbunden werden.

12. Verfahren gemäß einem der Ansprüche 9 bis 11, **dadurch gekennzeichnet, dass** die Superabsorberpartikel (12) mittels Hilfsverschweißung(en) (4) in der Position fixiert werden, wobei sich die Hilfsverschweißung(en) (4) je nach Menge der Absorbtion durch den Quelldruck des Superabsorbers (12) öffnen kann.

13. Verfahren gemäß einem der Ansprüche 9 bis 12, **dadurch gekennzeichnet, dass** die Superabsorberpartikel (12) mittels einer Präzisionsstreumaschine auf eine oder mehrere Materiallagen (10) flächig positioniert werden.

14. Verfahren gemäß einem der Ansprüche 9 bis 13, **dadurch gekennzeichnet, dass** die Breite und Länge der Materiallagen (10, 13) so gewählt wird, dass ein oder mehrere absorbierende Komposite (1) nebeneinander und eine Vielzahl Komposite (1) hintereinander gebildet werden, bei denen Bereiche ohne Superabsorber-Partikel aneinandergrenzen und die durch Zerschneiden innerhalb und entlang dieser Bereiche in Einzelkomposite (1) getrennt werden.
